# EUROPEAN PATENT SPECIFICATION

(11) **EP 0 271 271 B1**
(45) Date of publication and mention of the grant of the patent: **15.01.1992**
(21) Application number: 87310551.4
(22) Date of filing: 30.11.1987
(51) Int. Cl.: A61K 45/06, A61K 31/40

(54) **Pharmaceutical preparations**
Pharmazeutische Zubereitungen
Préparations pharmaceutiques

(30) Priority: 06.12.1986 GB 8629234; 24.12.1986 GB 8630926
(43) Date of publication of application: 15.06.1988
(73) Proprietor: BEECHAM GROUP PLC, Brentford, Middlesex TW8 9BD (GB)
(72) Inventor: Buckingham, Robin Edwin Beecham Pharmaceuticals, Harlow Essex CM19 5AD (GB)
(74) Representative: Tocher, Pauline

(56) References cited:
- EP-A- 0 173 848

## Description

The present invention relates to pharmaceutical preparations having antihypertensive activity.

EP-A-173848 describes the use of various benzopyran compounds, previously disclosed as antihypertensives, which are potassium channel activators useful in the treatment of cardiovascular disorders.

EP-A-205292 discloses a group of pyranopyridines which are also potassium channel activators, useful in the treatment of hypertension, cardiovascular disorders and disorders associated with smooth muscle contraction.

United Kingdom Patent No. 1489879 discloses the compound Nʺ-Cyano-N-4-pyridyl-Nʹ-1,2,2-trimethylpropylguanidine and, in Example 47, a process by which it can be prepared. The compound, which is referred to herein by its common name, pinacidil, is described in the patent as hypotensive compound. In "Drugs of the Future" Vol. VI(3), 149, 1981, pinacidil is described as a vasodilator. It is now known that pinacidil is a potassium channel activator.

Enalapril, Lisinopril and Captopril are angiotension converting enzyme (ACE) inhibitors having antihypertensive activity of formulae (A), (B) and (C): 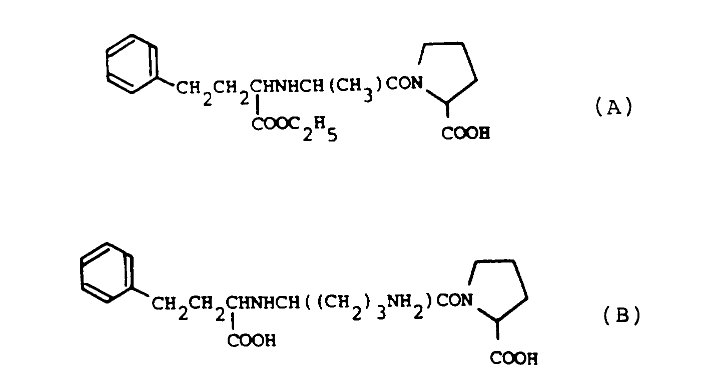 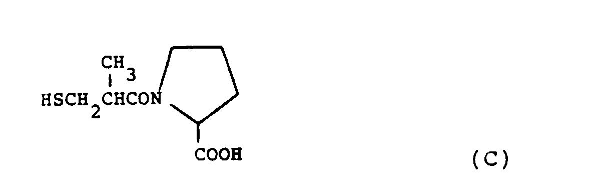
which are disclosed in Drugs, 31, (1986); Drugs Of the Future, 10(11), 948 (1985); and Drugs of the Future 6(11), 731 (1981), respectively.

It has now been found that a combination of a potassium channel activator antihypertensive agent, such as pinacidil or a benzopyran or pyranopyridine as hereinbefore referred to, and an ACE inhibitor antihypertensive agent, such as a compound of formula (A), (B) or (C), or a pharmaceutically acceptable salt of any of the foregoing has good antihypertensive activity. The effectiveness of the combination is greater than could be predicted from a consideration of the antihypertensive activities of the individual components and it appears that a synergistic effect is being produced.

Accordingly, the present invention provides a pharmaceutical product comprising a potassium channel activator antihypertensive agent, such as pinacidil or a compound of formula (I), or a pharmaceutically acceptable salt thereof: 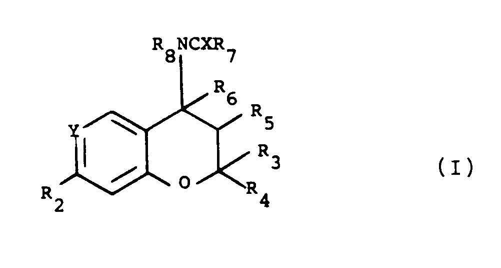
wherein
either Y is N and R₂ is hydrogen; or
Y is C-R₁
wherein
either one of R₁ and R₂ is hydrogen and the other is selected from the class of C₁₋₆ alkylcarbonyl, C₁₋₆ alkoxycarbonyl, C₁₋₆ alkylcarbonyloxy, C₁₋₆ alkylhydroxymethyl, nitro, cyano, chloro, trifluoromethyl, C₁₋₆ alkylsulphinyl, C₁₋₆ alkylsulphonyl, C₁₋₆ alkoxysulphinyl, C₁₋₆ alkoxysulphonyl, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkylthiocarbonyloxy, 1-mercapto C₂₋₇ alkyl, formyl, or aminosulphinyl, aminosulphonyl or aminocarbonyl, the amino moiety being optionally substituted by one or two C₁₋₆ alkyl groups, or C₁₋₆ alkylsulphinylamino, C₁₋₆ alkylsulphonylamino, C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂; or
one of R₁ and R₂ is nitro, cyano or C₁₋₃ alkylcarbonyl and the other is methoxy or amino optionally substituted by one or two C₁₋₆ alkyl or by C₂₋₇ alkanoyl;
either one of R₃ and R₄ is hydrogen or C₁₋₄ alkyl and the other is C₁₋₄ alkyl; or
R₃ and R₄ together are C₂₋₅ polymethylene;
either R₅ is hydrogen, hydroxy, C₁₋₆ alkoxy or C₁₋₇ acyloxy; and
R₆ is hydrogen; or
R₅ and R₆ together are a bond;
either R₇ is hydrogen, C₁₋₆ alkyl optionally substituted byhydroxy, C₁₋₆ alkoxy, C₁₋₆ alkoxycarbonyl or carboxy, C₁₋₆ alkyl substituted by halogen, or C₂₋₆ alkenyl; aryl or heteroaryl either being optionally substituted by one or more groups or atoms selected from the class of C₁₋₆ alkoxy, hydroxy, halogen, trifluoromethyl, nitro, cyano, C₁₋₁₂ carboxylic acyl, or amino or aminocarbonyl optionally substituted by one or two C₁₋₆ alkyl groups; and
R₈ is hydrogen or C₁₋₆ alkyl; or
R₇ and R₈ are joined together to form C₃₋₄ polymethylene or -CH₂-(CH₂)ₙ-Z-(CH₂)m- wherein m and n are integers 0 to 2 such that m+n is 1 or 2 and Z is oxygen, sulphur or NR₉ wherein R₉ is hydrogen, C₁₋₉ alkyl, C₂₋₇ alkanoyl, phenyl C₁₋₄ alkyl, naphthylcarbonyl, phenylcarbonyl or benzylcarbonyl optionally substituted in the phenyl or naphthyl ring by one or two C₁₋₆ alkyl, C₁₋₆ alkoxy or halogen; mono- or bi-cyclic heteroarylcarbonyl;
X is oxygen or sulphur; and
the R₈NCXR₇ moiety is trans to the R₅ group when R₅ is hydroxy, C₁₋₆ alkoxy or C₁₋₇ acyloxy;
and an ACE inhibitor antihypertensive agent, such as a compound of formula (A), (B) or (C): 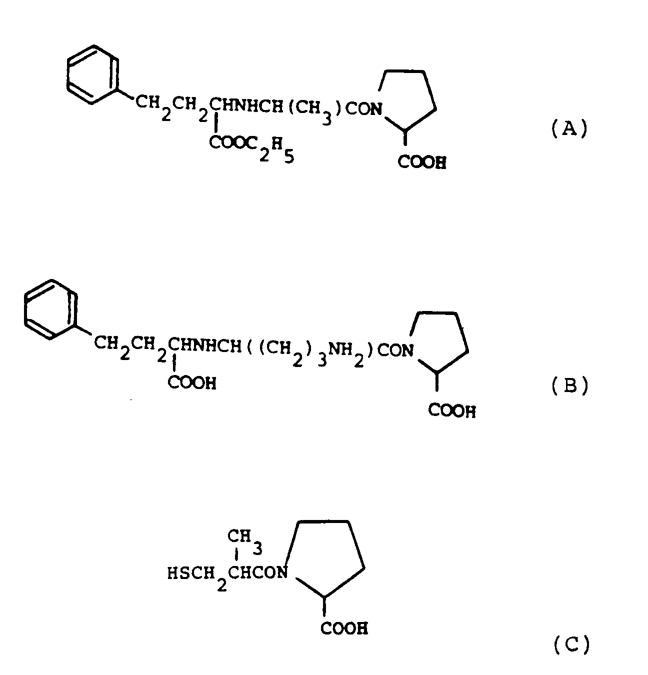
as a combined preparation for simultaneous, separate or sequential use in therapy of hypertension.

In a preferred aspect, the active components of the product are administered simultaneously.

The present invention further provides a pharmaceutical composition comprising a potassium channel activator antihypertensive agent, such as pinacidil or a compound of formula (I), or a pharmaceutically acceptable salt thereof and an ACE inhibitor antihypertensive agent, such as a compound of formula (A), (B) or (C); in combination with a pharmaceutically acceptable carrier.

The invention yet further provides the use of a potassium channel activator antihypertensive agent, such as pinacidil or a compound of formula (I) or a pharmaceutically acceptable salt thereof and an ACE inhibitor antihypertensive agent, such as a compound of formula (A), (B) or (C), in the manufacture of a pharmaceutical preparation for simultaneous, separate or sequential use in antihypertensive therapy.

Suitable and preferred values for the variable groups or atoms in formula (I) are as described for the corresponding variables in EP-A-76075, 91748, 93535, 95316, 107423, 120426, 120427, 126311, 126350, 126367, 138134 and 205292. Corresponding United States Patent references are U.S. Patent No's. 4446113, 4542149, 4510152, 4481214, 4496565, 4555509, 4610992, 4571406, 4629734, 4575511, 4677116 and U.S. Serial No. 871711, respectively.

A particularly preferred compound of formula (I) is the compound of Example 1 of EP-A-76075 and United States Patent No. 4446113,(±)-6-cyano-3,4-dihydro-2,2-dimethyl-trans-4-(2-oxo-1-pyrrolidinyl)-2H-benzo[b]pyran-3-ol.

Examples of pharmaceutically acceptable salts are as described in the aforementioned European Patent references.

The above described products and compositions have blood pressure lowering activity, and are potentially useful in the treatment of hypertension.

The compounds of formula (I), and salts thereof may be prepared as described in the aforementioned European and United States Patent references.

Pinacidil and salts thereof may be prepared as described in the aforementioned U.K. Patent.

The product of the invention may be administered by the oral route to humans and may be compounded in the form of syrup, tablets or capsule for either separate, sequential or simultaneous administration.

However, they may be adapted for other modes of administration, for example parenteral administration for patients suffering from heart failure. Other alternative modes of administration include sublingual or transdermal administration.

Generally, compositions containing from about 0.5 to 25 mg of a compound of formula (A), (B) or (C) per mg of pinacidil or a compound of formula (I) or a pharmaceutically acceptable salt thereof are effective, depending on the activity of the potassium channel activator.

In order to obtain consistency of administration it is preferred that a composition of the invention is in the form of a unit-dose. Suitable unit dose forms include tablets, capsules and powders in sachets or vials. Such unit dose forms may contain a total of from 0.5 to 100 mg of active compounds of the invention and more usually from 0.5 to 50 mg, for example 0.5 to 25 mg such as 0.5, 1, 2, 3, 5, 10, 15 or 20 mg. Such compositions may be administered from 1 to 6 times a day, more usually from 2 to 4 times a day, in a manner such that the daily dose is from 0.5 to 200 mg for a 70 kg human adult and more particularly fom 0.5 to 25 mg.

With the above indicated dosage range, no adverse toxicological effects are indicated with the compounds of the invention.

The compositions of the invention may be formulated with conventional excipients, such as a filler, a disintegrating agent, a binder, a lubricant, a flavouring agent. They are formulated in conventional manner, for example in a manner similar to that used for anti-hypertensive agents.

It is greatly preferred that the potassium channel activator antihypertensive agent, such as pinacidil or the compound of formula (I) or a pharmaceutically acceptable salt thereof and the ACE inhibitor antihypertensive agent, such as the compounds of formula (A), (B) or (C) are administered in the form of a unit-dose composition, such as a unit dose oral or parenteral composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling or tabletting. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

In addition such compositions may contain further active agents such as other classes of anti-hypertensive agents and diuretics.

As is common practice, the compositions will usually be accompanied by written or printed directions for use in the medical treatment concerned.

It will be appreciated that each component of the product of the invention may be administered by a different route.

The present invention yet further provides a method of treating hypertension in mammals including man, which comprises administering to the suffering mammal an anti-hypertensive effective amount of a pharmaceutical composition comprising a potassium channel activator antihypertensive agent, such as pinacidil or a compound of formula (I) or a pharmaceutically acceptable salt thereof and an ACE inhibitor antihypertensive agent, such as a compound of formula (A), (B) or (C), in combination with a pharmaceutically acceptable carrier.

The following pharmacological data illustrate the invention.
Compound 1 is 6-cyano-3,4-dihydro-2,2-dimethyl-trans-4-(2-oxo-1-pyrrolidinyl)-2H-benzo[b]pyran-3-ol (the compound of Example 1 of EP-A-76075 and US Patent No. 4446113);
Compound 2 is trans-3,4-dihydro-2,2-dimethyl-4-(2-oxopyrrolidin-1-yl)-2H-pyrano[3,2-c]pyridin-3-ol (the compound of Example 1 of EP-A-205292);
Compound 3 is 6-cyano-3,4-dihydro-2,2-dimethyl-trans-4-(N-3-methyltetrahydro-2-pyrimidone)-2H-benzo[b]pyran-3-ol, the compound of Example 12 of EP-A-107423 and US Patent No. 4496565);
Compound 4 is trans-4-N-acetylamino-6-cyano-3,4-dihydro-2,2-dimethyl-2H-benzo[b]pyran-3-ol, (the compound of Example 2 of EP-A-95316 and US Patent No. 4481214).

Potentiation of the anti-hypertensive response to Compounds of formula (I) has been observed in conscious male spontaneously hypertensive rats (SHR) when the compounds were combined with the angiotensin I converting enzyme inhibitor enalapril.

For the purpose of the experiment 36 rats were allotted at random to 4 groups, each of 9 rats. Systolic blood pressure (SBP) was measured non-invasively using an inflatable tail cuff technique as described in the aforementioned European Patent references. The protocol of the experiment was as follows:

| TIME (h) | GROUP 1 | GROUP 2 | GROUP 3 | GROUP 4 |
|---|---|---|---|---|
| 0 | Vehicle (water) 5 ml/kg po | Enalapril 3 mg/kg po | Vehicle 5 ml/kg po | Enalapril 3 mg/kg po |
| 0.75 | Vehicle (water) | Vehicle 5 ml/kg po | Compound 1 0.3 mg/kg po | Compound 1 0.3 mg/kg po |

SBP was measured in each rat prior to the treatments detailed above and then again at 1.75, 2.75 and 4.75 h. The results were as shown in Table I.

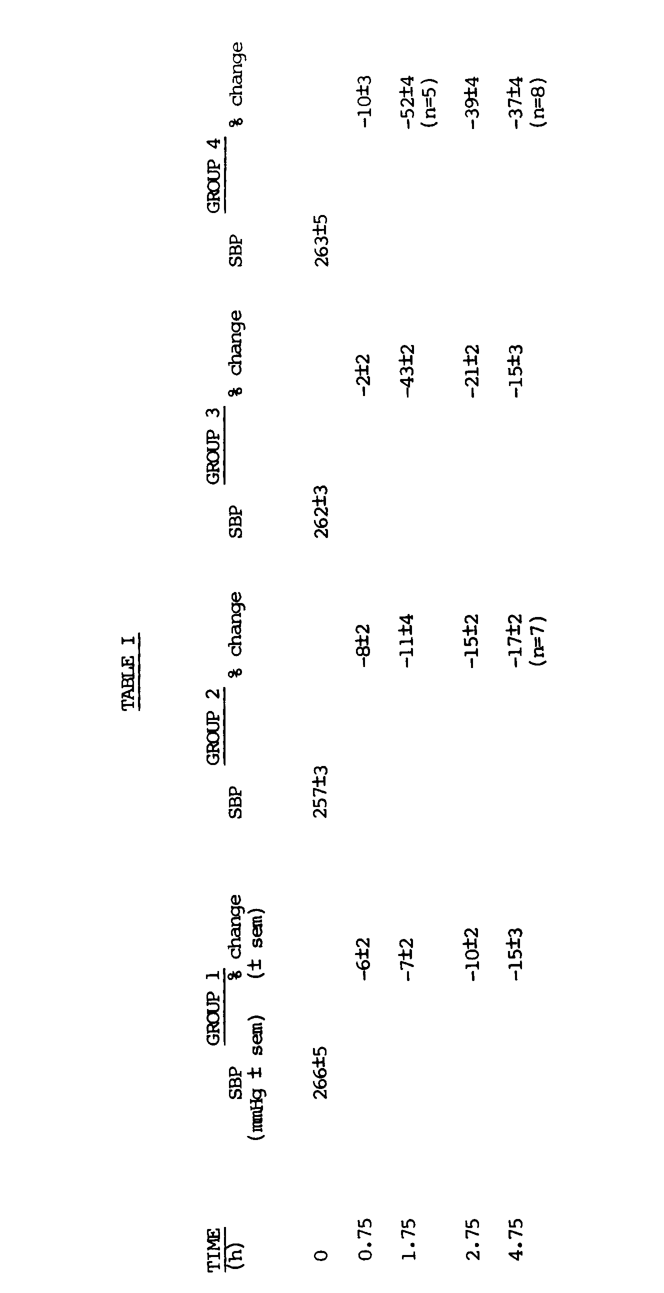

The results for compounds 2 to 4 were as follows:

| COMPOUND 2 | | | | |
|---|---|---|---|---|
| TIME | CONTROL | COMPOUND 2 0.6 mg/kg po | COMPOUND (A) 3 mg/kg po | COMPOUNDS 2 + (A) |
| 0 | 246±6 | 244±6 | 247±3 | 246±4 |
| 0.75 | -3±2 | 1±2 | -9±1 | -8±1 |
| 1.75 | -4±2 | -20±2 | -22±2 | -45±5 |
| 2.75 | -2±3 | -12±3 | -14±3 | -32±4 |
| 4.75 | -8±2 | -11±2 | -16±2 | -31±3 |

| COMPOUND 3 | | | | |
|---|---|---|---|---|
| TIME | CONTROL | COMPOUND 3 2.0 mg/kg po | COMPOUND (A) 3 mg/kg po | COMPOUNDS 3 + (A) |
| 0 | 251±5 | 253±6 | 254±4 | 255±3 |
| 0.75 | 0±2 | 0±3 | -8±2 | -13±2 |
| 1.75 | 0±2 | -20±3 | -10±3 | -44±5 |
| 2.75 | 2±2 | -22±3 | -12±4 | -48±4 |
| 4.75 | -6±3 | -21±3 | -21±3 | -48±3 |

| COMPOUND 4 | | | | |
|---|---|---|---|---|
| TIME | CONTROL | COMPOUND 4 0.15 mg/kg po | COMPOUND (A) 3 mg/kg po | COMPOUNDS 4 + (A) |
| 0 | 275±4 | 281±5 | 280±5 | 285±1 |
| 0.75 | -2±1 | 0±2 | -4±2 | -6±2 |
| 1.75 | -6±2 | -26±4 | -9±2 | -42±4(7) |
| 2.75 | -6±1 | -23±4 | -12±3 | -41±2 |
| 4.75 | -2±1 | -13±2 | -14±2 | -32±2 |

The protocol of the experiment for pinacidil was as follows:

| TIME (h) | GROUP 1 | GROUP 2 | GROUP 3 | GROUP 4 |
|---|---|---|---|---|
| 0 | Vehicle^{*} 5 ml/kg po | Enalapril 3 mg/kg po | Vehicle 5 ml/kg po | Enalapril 3 mg/kg po |
| 0.75 | Vehicle^{*} | Vehicle 5 ml/kg po | Pinacidil 1 mg/kg po | Pinacidil 1 mg/kg po |

| | | | | |
|---|---|---|---|---|
| *1% methylcellulose in water | | | | |

SBP was measured in each rat prior to the treatments detailed above and then again at 1.75, 2.75 and 4.75 h. The results were as shown in Table II.

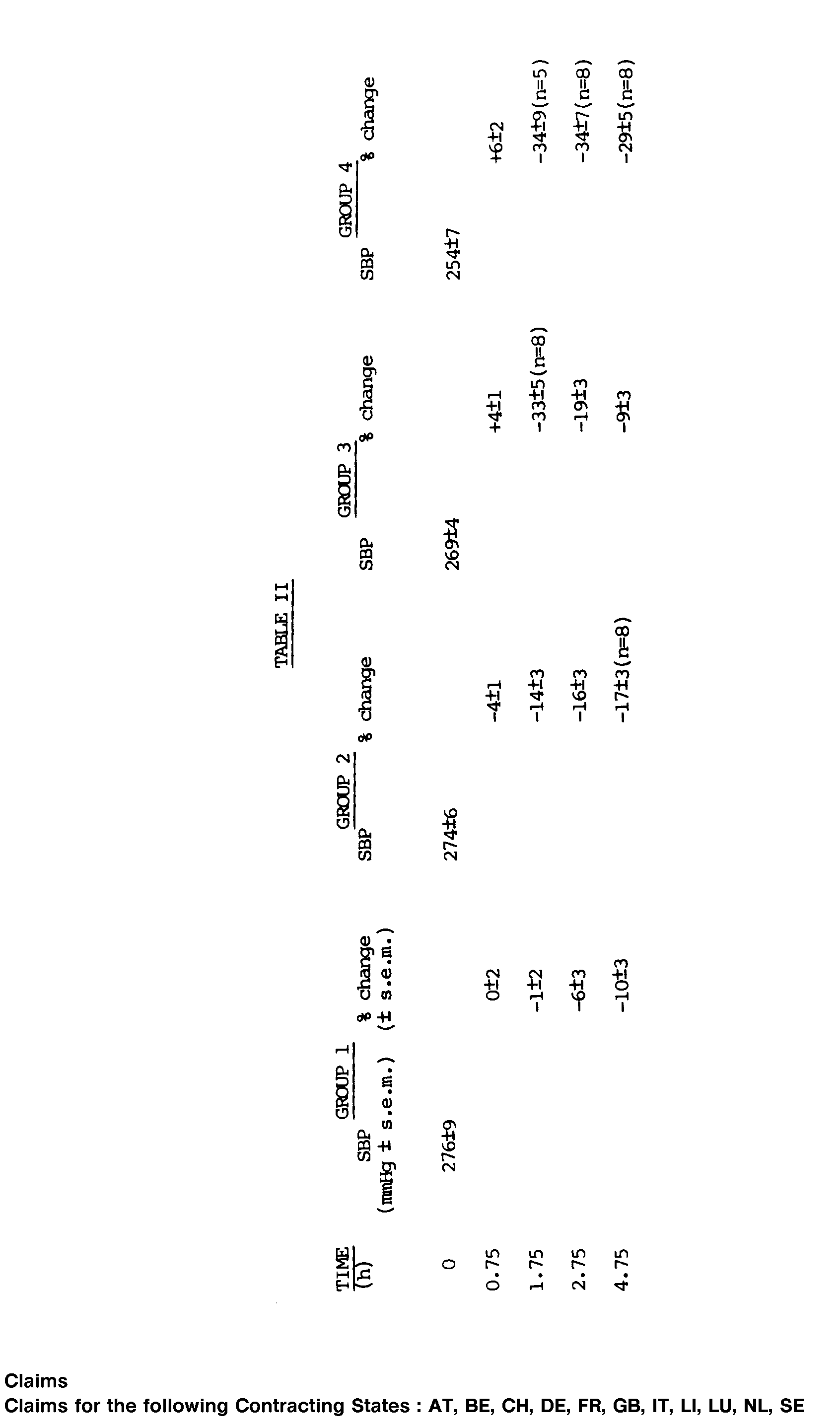

## Claims (Claims for the following Contracting State(s): AT, BE, CH, DE, FR, GB, IT, LI, LU, NL, SE)

1. A pharmaceutical product comprising a potassium channel activator antihypertensive agent and an ACE inhibitor antihypertensive agent as a combined preparation for simultaneous, separate or sequential use in therapy of hypertension.

2. A product according to claim 1 wherein the potassium channel activator antihypertensive agent is pinacidil or a compound of formula (I), or a pharmaceutically acceptable salt thereof: 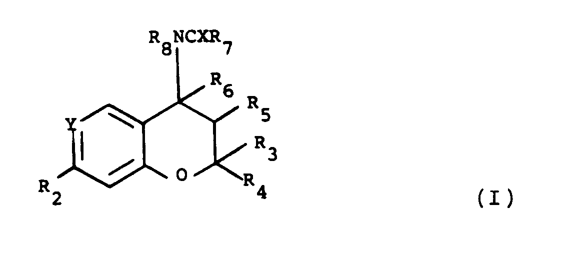 wherein
either Y is N and R₂ is hydrogen; or
Y is C-R₁
wherein
either one of R₁ and R₂ is hydrogen and the other is selected from the class of C₁₋₆ alkylcarbonyl, C₁₋₆ alkoxycarbonyl, C₁₋₆ alkylcarbonyloxy, C₁₋₆ alkylhydroxymethyl, nitro, cyano, chloro, trifluoromethyl, C₁₋₆ alkylsulphinyl, C₁₋₆ alkylsulphonyl, C₁₋₆ alkoxysulphinyl, C₁₋₆ alkoxysulphonyl, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkylthiocarbonyloxy, 1-mercapto C₂₋₇ alkyl, formyl, or aminosulphinyl, aminosulphonyl or aminocarbonyl, the amino moiety being optionally substituted by one or two C₁₋₆ alkyl groups, or C₁₋₆ alkylsulphinylamino, C₁₋₆ alkylsulphonylamino, C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂; or
one of R₁ and R₂ is nitro, cyano or C₁₋₃ alkylcarbonyl and the other is methoxy or amino optionally substituted by one or two C₁₋₆ alkyl or by C₂₋₇ alkanoyl;
either one of R₃ and R₄ is hydrogen or C₁₋₄ alkyl and the other is C₁₋₄ alkyl; or
R₃ and R₄ together are C₂₋₅ polymethylene;
either R₅ is hydrogen, hydroxy, C₁₋₆ alkoxy or C₁₋₇ acyloxy; and
R₆ is hydrogen; or
R₅ and R₆ together are a bond;
either R₇ is hydrogen, C₁₋₆ alkyl optionally substituted byhydroxy, C₁₋₆ alkoxy, C₁₋₆ alkoxycarbonyl or carboxy, C₁₋₆ alkyl substituted by halogen, or C₂₋₆ alkenyl; aryl or heteroaryl either being optionally substituted by one or more groups or atoms selected from the class of C₁₋₆ alkoxy, hydroxy, halogen, trifluoromethyl, nitro, cyano, C₁₋₁₂ carboxylic acyl, or amino or aminocarbonyl optionally substituted by one or two C₁₋₆ alkyl groups; and
R₈ is hydrogen or C₁₋₆ alkyl; or
R₇ and R₈ are joined together to form C₃₋₄ polymethylene or -CH₂-(CH₂)ₙ-Z-(CH₂)m- wherein m and n are integers 0 to 2 such that m+n is 1 or 2 and Z is oxygen, sulphur or NR₉ wherein R₉ is hydrogen, C₁₋₉ alkyl, C₂₋₇ alkanoyl, phenyl C₁₋₄ alkyl, naphthylcarbonyl, phenylcarbonyl or benzylcarbonyl optionally substituted in the phenyl or naphthyl ring by one or two C₁₋₆ alkyl, C₁₋₆ alkoxy or halogen; mono- or bi-cyclic heteroarylcarbonyl;
X is oxygen or sulphur; and
the R₈NCXR₇ moiety is trans to the R₅ group when R₅ is hydroxy, C₁₋₆ alkoxy or C₁₋₇ acyloxy;
and the ACE inhibitor antihypertensive agent, is a compound of formula (A), (B) or (C):

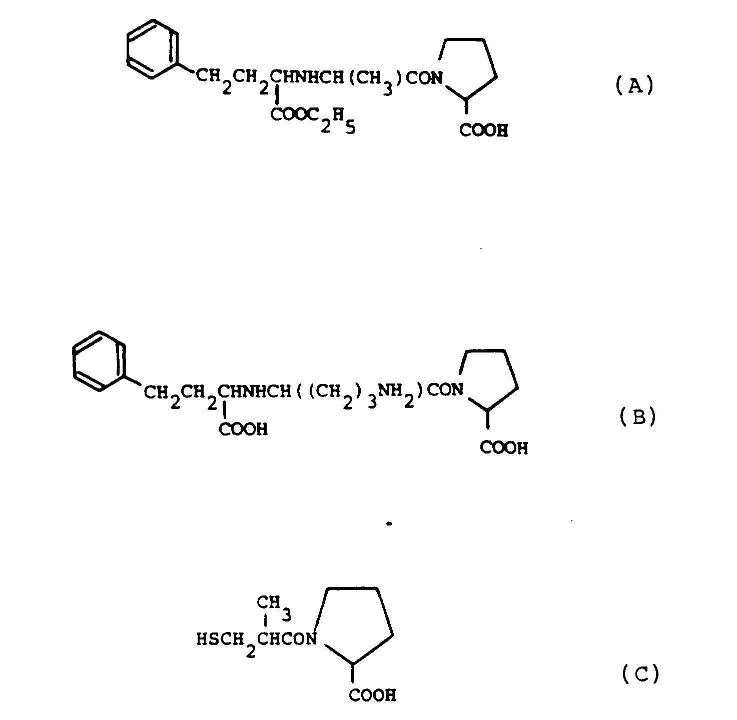

3. A product according to claim 2 wherein the potassium channel activator antihypertensive agent is of formula (I) wherein Y is N or C-R₁ wherein R₁ is nitro, cyano or C₁₋₃ alkylcarbonyl, and R₂ is hydrogen.

4. A product according to claim 2 or 3 wherein R₃ and R₄ are both methyl groups.

5. A product according to any one of claims 2, 3 or 4 wherein R₇ and R₈ are joined together to form C₃₋₄ polymethylene or -CH₂-(CH₂)ₙ-Z-(CH₂)ₘ- wherein m, n and Z are as defined in claim 2; or R₈ is hydrogen or methyl and R₇ is ethyl, methyl, hydrogen, or phenyl optionally substituted by one, two, three or four groups or atoms selected from methyl, methoxy, hydroxy, fluoro and chloro.

6. A product according to claim 5 wherein the compound of formula (I) is (±)-6-cyano-3,4-dihydro-2,2-dimethyl-trans-4-(2-oxo-1-pyrrolidinyl)-2H-benzo[b]pyran-3-ol.

7. A pharmaceutical composition comprising a potassium channel activator antihypertensive agent and an ACE inhibitor antihypertensive agent, as defined in any one of claims 1 to 6, in combination with a pharmaceutically acceptable carrier.

8. A composition according to claim 7, wherein the potassium channel activator antihypertensive agent is pinacidil or a compound of formula (I), as defined in claim 1, or a pharmaceutically acceptable salt thereof, and the ACE inhibitor antihypertensive agent is a compound of formula (A), (B) or (C), as defined in claim 1.

9. A composition according to claim 8, containing 0.5 to 25 mg of a compound of formula (A), (B), or (C) per mg of pinacidil or a compound of formula (I), or a pharmaceutically acceptable salt thereof.

10. The use of a product or pharmaceutical composition according to any one of claims 1 to 9, in the manufacture of a combined preparation for simultaneous, separate or sequential use in therapy of hypertension.

11. A product or pharmaceutical composition according to any one of claims 1 to 10, for use in the treatment of hypertension.

## Claims (Claims for the following Contracting State(s): ES, GR)

1. A process for the preparation of a pharmaceutical composition comprising a potassium channel activator antihypertensive agent and an ACE inhibitor antihypertensive agent, and a pharmaceutically acceptable carrier, which comprises the admixture of the active ingredients and the carrier at non-extreme temperature 0 to 100°C and non-extreme pressure 0.1 to 10 atmospheres, preferably ambient temperature and atmospheric pressure.

2. A process according to claim 1 wherein the potassium channel activator antihypertensive agent is pinacidil or a compound of formula (I), or a pharmaceutically acceptable salt thereof: 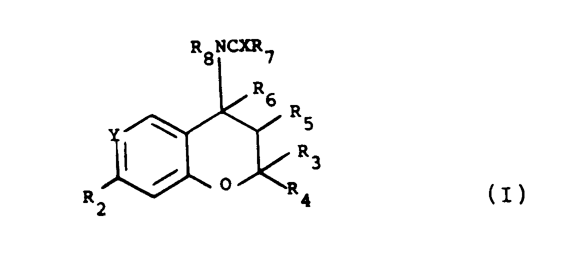 wherein
either Y is N and R₂ is hydrogen; or
Y is C-R₁
wherein
either one of R₁ and R₂ is hydrogen and the other is selected from the class of C₁₋₆ alkylcarbonyl, C₁₋₆ alkoxycarbonyl, C₁₋₆ alkylcarbonyloxy, C₁₋₆ alkylhydroxymethyl, nitro, cyano, chloro, trifluoromethyl, C₁₋₆ alkylsulphinyl, C₁₋₆ alkylsulphonyl, C₁₋₆ alkoxysulphinyl, C₁₋₆ alkoxysulphonyl, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkylthiocarbonyloxy, 1-mercapto C₂₋₇ alkyl, formyl, or aminosulphinyl, aminosulphonyl or aminocarbonyl, the amino moiety being optionally substituted by one or two C₁₋₆ alkyl groups, or C₁₋₆ alkylsulphinylamino, C₁₋₆ alkylsulphonylamino, C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂; or
one of R₁ and R₂ is nitro, cyano or C₁₋₃ alkylcarbonyl and the other is methoxy or amino optionally substituted by one or two C₁₋₆ alkyl or by C₂₋₇ alkanoyl;
either one of R₃ and R₄ is hydrogen or C₁₋₄ alkyl and the other is C₁₋₄ alkyl; or
R₃ and R₄ together are C₂₋₅ polymethylene;
either R₅ is hydrogen, hydroxy, C₁₋₆ alkoxy or C₁₋₇ acyloxy; and
R₆ is hydrogen; or
R₅ and R₆ together are a bond;
either R₇ is hydrogen, C₁₋₆ alkyl optionally substituted byhydroxy, C₁₋₆ alkoxy, C₁₋₆ alkoxycarbonyl or carboxy, C₁₋₆ alkyl substituted by halogen, or C₁₋₆ alkenyl; aryl or heteroaryl either being optionally substituted by one or more groups or atoms selected from the class of C₁₋₆ alkoxy, hydroxy, halogen, trifluoromethyl, nitro, cyano, C₁₋₁₂ carboxylic acyl, or amino or aminocarbonyl optionally substituted by one or two C₁₋₆ alkyl groups; and
R₈ is hydrogen or C₁₋₆ alkyl; or
R₇ and R₈ are joined together to form C₃₋₄ polymethylene or -CH₂-(CH₂)ₙ-Z-(CH₂)m- wherein m and n are integers 0 to 2 such that m+n is 1 or 2 and Z is oxygen, sulphur or NR₉ wherein R₉ is hydrogen, C₁₋₉ alkyl, C₂₋₇ alkanoyl, phenyl C₁₋₄ alkyl, naphthylcarbonyl, phenylcarbonyl or benzylcarbonyl optionally substituted in the phenyl or naphthyl ring by one or two C₁₋₆ alkyl, C₁₋₆ alkoxy or halogen; mono- or bi-cyclic heteroarylcarbonyl
X is oxygen or sulphur; and
the R₈NCXR₇ moiety is trans to the R₅ group when R₅ is hydroxy, C₁₋₆ alkoxy or C₁₋₇ acyloxy;
and the ACE inhibitor antihypertensive agent, is a compound of formula (A), (B) or (C):

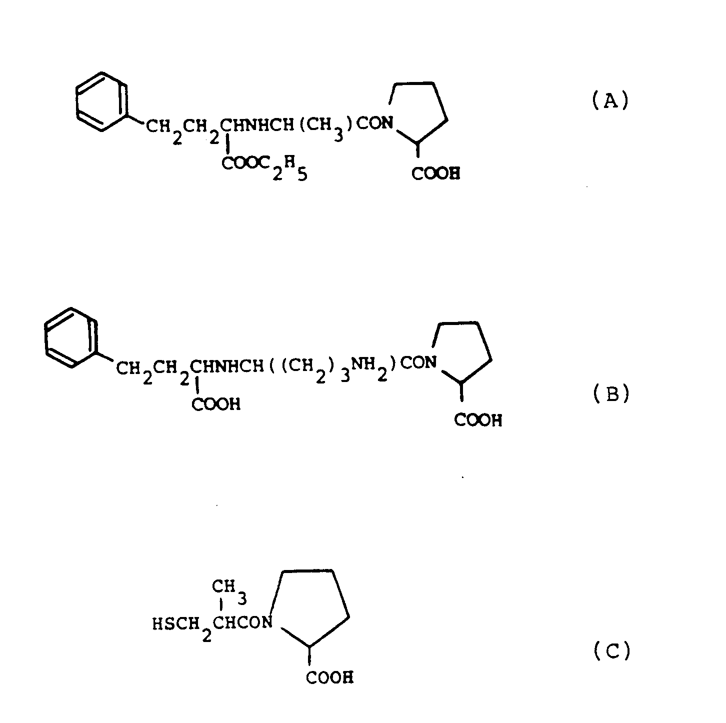

3. A process according to claim 2 wherein the potassium channel activator antihypertensive agent is of formula (I) wherein Y is N or C-R₁ wherein R₁ is nitro, cyano or C₁₋₃ alkylcabonyl, and R₂ is hydrogen.

4. A process according to claim 2 or 3 wherein R₃ and R₄ are both methyl groups.

5. A process according to any one of claims 2, 3 or 4 wherein R₇ and R₈ are joined together to form C₃₋₄ polymethylene or -CH₂-(CH₂)-2-(CH₂)ₘ- wherein m, n and Z are as defined in claim 1; or R8 is hydrogen or methyl and R₇ is ethyl, methyl, hydrogen, or phenyl optionally substituted by one, two, three or four groups or atoms selected from methyl, methoxy, hydroxy, fluoro and chloro.

6. A process according to claim 5 wherein the compound of formula (I) is (±)-6-cyano-3,4-dihydro-2,2-dimethyl-trans-4-(2-oxo-1-pyrrolidinyl)-2H-benzo[b]pyran-3-ol.

7. A process according to claim 2, wherein the composition contains 0.5 to 25 mg of a compound of formula (A), (B), or (C) per mg of pinacidil or a compound of formula (I), or a pharmaceutically acceptable salt thereof.

8. The use of a pharmaceutical product comprising a potassium channel activator antihypertensive agent and an ACE inhibitor antihypertensive agent, in the manufacture of a combined preparation for simultaneous, separate or sequential use in therapy of hypertension.

## Revendications (Revendications pour l'(les) Etat(s) contractant(s) suivant(s): AT, BE, CH, DE, FR, GB, IT, LI, LU, NL, SE)

1. Produit pharmaceutique caractérisé en ce qu'il comprend un agent anti-hypertenseur activateur des canaux de potassium et un agent anti-hypertenseur inhibiteur d'ACE sous forme d'une préparation combinée à utiliser de façon simultanée, séparée ou successive dans le traitement de l'hypertension.

2. Produit suivant la revendication 1, caractérisé en ce que l'agent anti-hypertenseur activateur des canaux de potassium est une pinacidile ou un composé de formule (I) , ou un sel de ceux-ci acceptable du point de vue pharmaceutique : 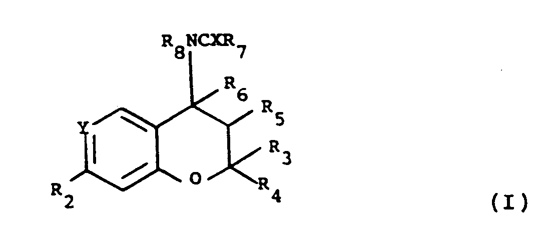 dans laquelle
soit Y est un atome d'azote et R₂ est un atome d'hydrogène ;
soit Y est un radical C-R₁
où soit l'un des R₁ et R₂ est un atome d'hydrogène et l'autre est choisi dans la classe comprenant un groupe alkyl-(C₁₋₆)carbonyle, alcoxy(C₁₋₆)carbonyle, alkyl(C₁₋₆)carbonyloxy, alkyl(C₁₋₆) hydroxyméthyle, nitro, cyano, un atome de chlore, un groupe trifluorométhyle, alkyl(C₁₋₆)-sulfinyle, alkyl(C₁₋₆)sulfonyle, alcoxy(C₁₋₆)sulfinyle, alcoxy(C₁₋₆)sulfonyle, alkyl(C₁₋₆)carbonylamino, alcoxy(C₁₋₆)carbonylamino, alkyl(C₁₋₆)thiocarbonyle, alcoxy(C₁₋₆)thiocarbonyle, alkyl(C₁₋₆)thiocarbonyloxy, 1-mercaptoalkyle en C₂₋₇ , formyle ou aminosulfinyle, aminosulfonyle ou aminocarbonyle, la partie amino étant éventuellement substituée par un ou deux groupes alkyles en C₁₋₆ ou un groupe alkyl(C₁₋₆)sulfinylamino, alkyl(C₁₋₆)sulfonylamino, alcoxy(C₁₋₆)sulfonylamino ou alcoxy(C₁₋₆)sulfonylamino, ou éthylényle substitué en position terminale par un groupe alkyl(C₁₋₆)carbonyle, nitro ou cyano, ou -C(alkyle en C₁₋₆)NOH ou -C(alkyle en C₁₋₆)NNH₂ ; soit
l'un des R₁ et R₂ est un groupe nitro, cyano ou alkylcarbonyle et l'autre est un groupe méthoxy ou amino éventuellement substitué par un ou deux groupes alkyles en C₁₋₆ ou par un groupe alcanoyle en C₂₋₇ ;
soit l'un des R₃ et R₄ est un atome d'hydrogène ou un groupe alkyle en C₁₋₄ et l'autre est un groupe alkyle en C₁₋₄ ; soit
R₃ et R₄ forment ensemble un radical polyméthylène en C₂₋₅ ;
soit R₅ est un atome d'hydrogène, un groupe hydroxy, alcoxy en C₁₋₆ ou acyloxy en C₁₋₇ ; et
R₆ est un atome d'hydrogène ; soit
R₅ et R₆ forment ensemble une liaison ;
soit R₇ est un atome d'hydrogène, un groupe alkyle en C₁₋₆ éventuellement substitué par un groupe hydroxy, alcoxy en C₁₋₆ alcoxy(C₁₋₆)carbonyle ou carboxy, alkyle en C₁₋₆ substitué par un atome d'halogène, ou un groupe alcényle en C₂₋₆ ; un groupe aryle ou hétéroaryle l'un ou l'autre étant éventuellement substitué par un ou plusieurs groupes ou atomes choisis dans la classe comprenant un groupe alcoxy en C₁-₆ , hydroxy, un atome d'halogène, un groupe trifluorométhyle, nitro, cyano, acylcarboxylique en C₁₋₁₂ ou amino ou aminocarbonyle éventuellement substitué par un ou deux groupes alkyles en C₁₋₆ ; et
R₈ est un atome d'hydrogène ou un groupe alkyle en C₁₋₆ ; soit
R₇ et R₈ sont réunis ensemble pour former un radical polyméthylène en C₃₋₄ ou -CH₂-(CH₂)ₙ-Z-(CH₂)ₘ- dans lequel m et n sont des entiers de 0 à 2 tel que m + n = 1 ou 2 et Z est un atome d'oxygène, de soufre ou un radical NR₉ dans lequel R₉ est un atome d'hydrogène, un groupe alkyle en C₁₋₉ , alcanoyle en C₂₋₇ , phényl-alkyle en c₁₋₄ , naphtylcarbonyle, phénylcarbonyle ou benzylcarbonyle dont le cycle phényle ou naphtyle est éventuellement substitué par un ou deux groupes alkyles en C₁₋₆ , alcoxy en C₁₋₆ ou un atome d'halogène ; groupe hétéroarylcarbonyle mono- ou bi-cyclique ;
X est un atome d'oxygène ou de soufre ; et
la partie R₈NCXR₇ est trans par rapport au groupe R₅ lorsque R₅ est un groupe hydroxy, alcoxy en C₁₋₆ ou acyloxy en C₁₋₇ ;
et en ce que l'agent anti-hypertenseur inhibiteur d'ACE est un composé de formule (A), (B) ou (C) :

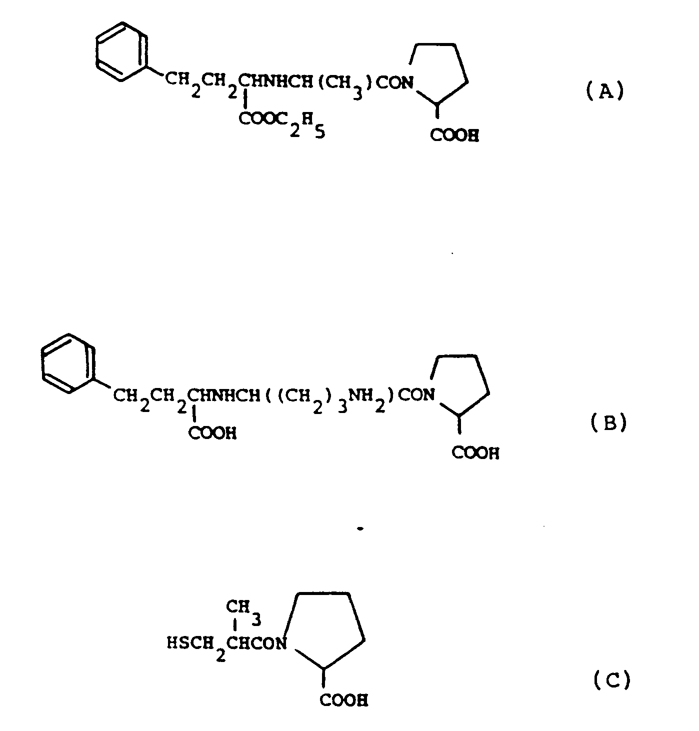

3. Produit suivant la revendication 2, caractérisé en ce que l'agent anti-hypertenseur activateur des canaux de potassium est représenté par la formule (I) dans laquelle Y est un atome d'azote ou un radical C-R₁ où R₁ est un groupe nitro, cyano ou alkyl(C₁₋₃)carbonyle et R₂ est un atome d'hydrogène.

4. Produit suivant la revendication 2 ou la revendication 3, caractérisé en ce que R₃ et R₄ sont tous deux des groupes méthyles.

5. Produit suivant l'une quelconque des revendications 2, 3 ou 4, caractérisé en ce que R₇ et R₈ sont réunis pour former un radical polyméthylène en C₂₋₄ ou -CH₂(CH₂)ₙ-Z-(CH₂)ₘ- dans lequel m, n et Z sont tels que définis dans la revendication 2 ; ou bien R₈ est un atome d'hydrogène ou un groupe méthyle et R₇ est un groupe éthyle, méthyle, un atome d'hydrogène ou un groupe phényle éventuellement substitué par un, deux, trois ou quatre groupes ou atomes choisis parmi les groupes méthyle, méthoxy, hydroxy et atomes de fluor et de chlore.

6. Produit suivant la revendication 5, caractérisé en ce que le composé de formule (i) est le (+)-6-cyano-3,4-dihydro-2,2-diméthyl-trans-4-(2-oxo-1-pyrrolidinyl)-2H)-benzo/b/pyran-3-ol.

7. Composition pharmaceutique, caractérisée en ce qu'elle comprend un agent anti-hypertenseur activateur des canaux de potassium et un agent anti-hypertenseur inhibiteur d'ACE tels que définis dans l'une quelconque des revendications 1 à 6, en association avec un support acceptable du point de vue pharmaceutique.

8. Composition suivant la revendication 7, caractérisée en ce que l'agent anti-hypertenseur activateur des canaux de potassium est la pinacidile ou un composé de formule (I) tels que définis dans la revendication 1, ou un sel de ceux-ci acceptable du point de vue pharmaceutique, et en ce que l'agent anti-hypertenseur inhibiteur d'ACE est un composé de formule (A), (B) ou (C) tel que défini dans la revendication 2.

9. Composition suivant la revendication 8, caractérisée en ce qu'elle contient 0,5 à 25 mg d'un composé de formule (A), (B) ou (C) par mg de pinacidile ou d'un composé de formule (I) ou d'un sel de ceux-ci acceptable du point de vue pharmaceutique.

10. Utilisation d'un produit ou d'une composition pharmaceutique suivant l'une quelconque des revendications 1 à 9 dans la fabrication d'une préparation combinée à utiliser de façon simultanée, séparée ou successive dans le traitement de l'hypertension.

11. Produit ou composition pharmaceutique suivant l'une quelconque des revendications 1 à 10 utile dans le traitement de l'hypertension.

## Revendications (Revendications pour l'(les) Etat(s) contractant(s) suivant(s): ES, GR)

1. Procédé pour la préparation d'une composition pharmaceutique comprenant un agent anti-hypertenseur activateur des canaux de potassium et un agent anti-hypertenseur inhibiteur d'ACE ainsi qu'un support acceptable du point de vue pharmaceutique, caractérisé en ce qu'il comprend le mélange des composants actifs et du support à une température non-extrême comprise entre 0 et 100°C et sous une pression non-extrême, comprise entre 0,1 et 10 atmosphères, de préférence à la température ambiante et à la pression atmosphérique.

2. Procédé suivant la revendication 1, caractérisé en ce que l'agent anti-hypertenseur activateur des canaux de potassium est une pinacidile ou un composé de formule (I) , ou un sel de ceux-ci acceptable du point de vue pharmaceutique : 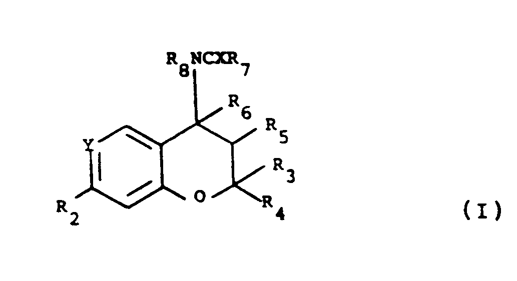 dans laquelle
soit Y est un atome d'azote et R₂ est un atome d'hydrogène ;
soit Y est un radical C-R₁
où soit l'un des R₁ et R₂ est un atome d'hydrogène et l'autre est choisi dans la classe comprenant un groupe alkyl(C₁₋₆)carbonyle, alcoxy(C₁₋₆)carbonyle, alkyl(C₁₋₆)carbonyloxy, alkyl(C₁₋₆) hydroxyméthyle, nitro, cyano, un atome de chlore, un groupe trifluorométhyle, alkyl(C₁₋₆)-sulfinyle, alkyl(C₁₋₆)sulfonyle, alcoxy(C₁₋₆)sulfinyle, alcoxy(C₁₋₆)sulfonyle, alkyl(C₁₋₆)carbonylamino, alcoxy(C₁₋₆)carbonylamino, alkyl(C₁₋₆)thiocarbonyle, alcoxy(C₁₋₆)thiocarbonyle, alkyl(C₁₋₆)thiocarbonyloxy, 1-mercaptoalkyle en C₂₋₇ , formyle ou aminosulfinyle, aminosulfonyle ou aminocarbonyle, la partie amino étant éventuellement substituée par un ou deux groupes alkyles en C₁₋₆ ou un groupe alkyl(C₁₋₆)sulfinylamino, alkyl(C₁₋₆)sulfonylamino, alcoxy(C₁₋₆)sulfinylamino ou alcoxy(C₁₋₆)sulfonylamino, ou éthylényle substitué en position terminale par un groupe alkyl(C₁₋₆)carbonyle, nitro ou cyano, ou -C(alkyle en C₁₋₆)NOH ou -C(alkyle en C₁₋₆)NNH₂ ; soit
l'un des R₁ et R₂ est un groupe nitro, cyano ou alkyl(C₁₋₃)carbonyle et l'autre est un groupe méthoxy ou amino éventuellement substitué par un ou deux groupes alkyles en C₁₋₆ ou par un groupe alcanoyle en C₂₋₇ ;
soit l'un des R₃ et R₄ est un atome d'hydrogène ou un groupe alkyle en C₁₋₄ et l'autre est un groupe alkyle en C₁₋₄ ; soit
R₃ et R₄ forment ensemble un radical polyméthylène en C₂₋₅ ;
soit R₅ est un atome d'hydrogène, un groupe hydroxy, alcoxy en C₁₋₆ ou acyloxy en C₁₋₇ ; et
R₆ est un atome d'hydrogène ; soit
R₅ et R₆ forment ensemble une liaison ;
soit R₇ est un atome d'hydrogène, un groupe alkyle en C₁₋₆ éventuellement substitué par un groupe hydroxy, alcoxy en C₁₋₆, alcoxy(C₁₋₆)carbonyle ou carboxy, alkyle en C₁₋₆ substitué par un atome d'halogène, ou un groupe alcényle en C₂₋₆ ; un groupe aryle ou hétéroaryle l'un ou l'autre étant éventuellement substitué par un ou plusieurs groupes ou atomes choisis dans la classe comprenant un groupe alcoxy en C₁₋₆, hydroxy, un atome d'halogène, un groupe trifluorométhyle, nitro, cyano, acylcarboxylique en C₁₋₁₂ ou amino ou aminocarbonyle éventuellement substitué par un ou deux groupes alkyles en C₁₋₆ ; et
R₈ est un atome d'hydrogène ou un groupe alkyle en C₁₋₆ ; soit
R₇ et R₈ sont réunis ensemble pour former un radical polyméthylène en C₃₋₄ ou -CH₂-(CH₂)ₙ-Z-(CH₂)ₘ- dans lequel m et n sont des entiers de 0 à 2 tel que m + n = 1 ou 2 et Z est un atome d'oxygène, de soufre ou un radical NR₉ dans lequel R₉ est un atome d'hydrogène, un groupe alkyle en C₁₋₉, alcanoyle en c₂₋₇, phényl-alkyle en C₁₋₄, naphtylcarbonyle, phénylcarbonyle ou benzylcarbonyle dont le cycle phényle ou naphtyle est éventuellement substitué par un ou deux groupes alkyles en C₁₋₆, alcoxy en C₁₋₆ ou un atome d'halogène ; groupe hétéroarylcarbonyle mono- ou bi-cyclique ;
X est un atome d'oxygène ou de soufre ; et
la partie R₈NCXR₇ est trans par rapport au groupe R₅ lorsque R₅ est un groupe hydroxy, alcoxy en C₁₋₆ ou acyloxy en C₁₋₇ ;
et en ce que l'agent anti-hypertenseur inhibiteur d'ACE est un composé de formule (A), (B) ou (C) :

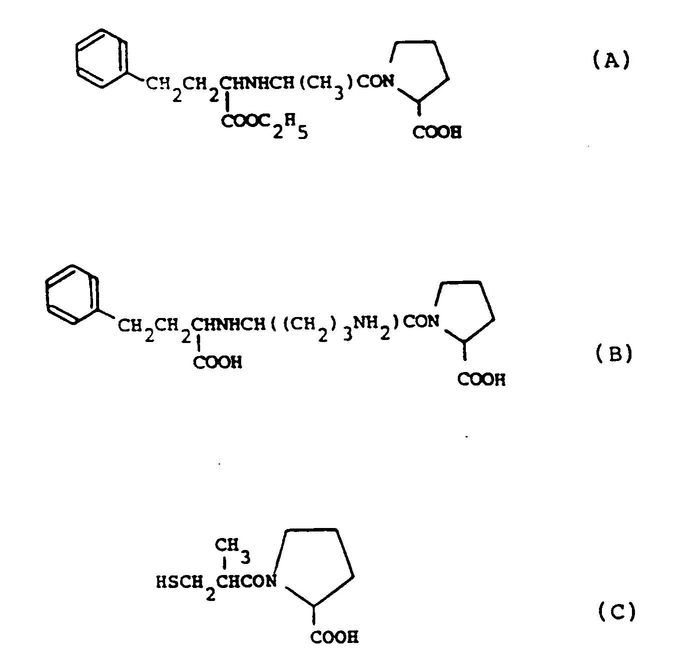

3. Procédé suivant la revendication 2, caractérisé en ce que l'agent anti-hypertenseur activateur des canaux de potassium est représenté par la formule (I) dans laquelle Y est un atome d'azote ou un radical C-R₁ où R₁ est un groupe nitro, cyano ou alkyl(C₁₋₃)carbonyle et R₂ est un atome d'hydrogène.

4. Procédé suivant la revendication 2 ou la revendication 3, caractérisé en ce que R₃ et R₄ sont tous deux des groupes méthyles.

5. Procédé suivant l'une quelconque des revendications 2, 3 ou 4, caractérisé en ce que R₇ et R₈ sont réunis pour former un radical polyméthylène en C₂₋₄ ou -CH₂-(CH₂)ₙ-Z-(CH₂)ₘ- dans lequel m, n et Z sont tels que définis dans la revendication 2 ; ou bien R₈ est un atome d'hydrogène ou un groupe méthyle et R₇ est un groupe éthyle, méthyle, un atome d'hydrogène ou un groupe phényle éventuellement substitué par un, deux, trois ou quatre groupes ou atomes choisis parmi les groupes méthyle, méthoxy, hydroxy et atomes de fluor et de chlore.

6. Procédé suivant la revendication 5, caractérisé en ce que le composé de formule (i) est le (±)-6-cyano-3,4-dihydro-2,2-diméthyl-trans-4-(2-oxo-1-pyrrolidinyl)-2H)-benzo/b/pyran-3-ol.

7. Procédé suivant la revendication 2, caractérisé en ce que la composition contient de 0,5 à 25 mg d'un composé de formule (A), (B) ou (C) par mg de pinacidile ou d'un composé de formule (I), ou d'un sel de ceux-ci acceptable du point de vue pharmaceutique.

8. Utilisation d'un produit pharmaceutique comprenant un agent anti-hypertenseur activateur des canaux de potassium et un agent anti-hypertenseur inhibiteur d'ACE, dans la fabrication d'une préparation combinée à utiliser de façon simultanée, séparée ou successive dans le traitement de l'hypertension.

## Patentansprüche (Patentansprüche für folgende(n) Vertragsstaat(en): AT, BE, CH, DE, FR, GB, IT, LI, LU, NL, SE)

1. Pharmazeutisches Erzeugnis, umfassend ein Kaliumkanalaktivator-Antibluthochdruckmittel und ein ACE-Inhibitor-Antibluthochdruckmittel als Kombinationspräparat zur gleichzeitigen, getrennten oder aufeinanderfolgenden Verwendung bei der Bluthochdrucktherapie.

2. Erzeugnis nach Anspruch 1, wobei das Kaliumkanalaktivator-Antibluthochdruckmittel Pinacidil oder eine Verbindung der Formel (I) ist oder ein pharmazeutisch verträgliches Salz davon, 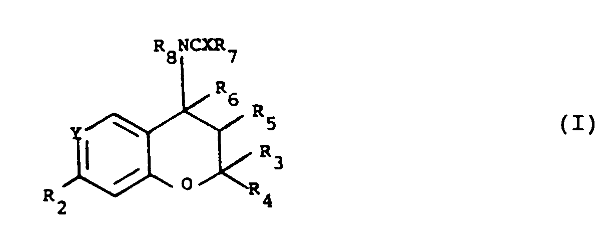 in der
entweder Y ein Stickstoffatom und R₂ ein Wasserstoffatom ist; oder
Y die Gruppe C-R₁ bedeutet,
wobei entweder einer der Reste R₁ und R₂ ein Wasserstoffatom ist und der andere einen C₁₋₆-Alkylcarbonyl-, C₁₋₆-Alkoxycarbonyl-, C₁₋₆-Alkylcarbonyloxy-, C₁₋₆-Alkylhydroxymethyl-, Nitro-, Cyano-, Chlor-, Trifluormethyl-, C₁₋₆-Alkylsulfinyl-, C₁₋₆-Alkylsulfonyl-, C₁₋₆-Alkoxysulfinyl-, C₁₋₆-Alkoxysulfonyl-, C₁₋₆-Alkylcarbonylamino-, C₁₋₆-Alkoxycarbonylamino-, C₁₋₆-Alkylthiocarbonyl-, C₁₋₆-Alkoxythiocarbonyl-, C₁₋₆-Alkylthiocarbonyloxy-, 1-Mercapto-C₂₋₇-alkyl-, Formyl- oder Aminosulfinyl-, Aminosulfonyl- oder Aminocarbonylrest, wobei die Aminogruppe gegebenenfalls mit einem oder zwei C₁₋₆-Alkylresten substituiert ist, oder einen C₁₋₆-Alkylsulfinylamino-, C₁₋₆-Alkylsulfonylamino-, C₁₋₆-Alkoxysulfinylamino- oder C₁₋₆-Alkoxysulfonylaminorest oder einen endständig mit einer C₁₋₆-Alkylcarbonyl-, Nitro- oder Cyanogruppe substituierten Ethylenylrest oder die Gruppe -C(C₁₋₆-Alkyl)NOH oder -C(C₁₋₆-Alkyl)NNH₂ bedeutet;
oder einer der Reste R₁ und R₂ eine Nitro-, Cyano- oder C₁₋₃-Alkylcarbonylgruppe bedeutet und der andere eine Methoxy- oder eine gegebenenfalls mit einem oder zwei C₁₋₆-Alkyl- oder mit einem C₂₋₇-Alkanoylrest substituierte Aminogruppe ist;
entweder einer der Reste R₃ und R₄ Wasserstoff oder einen C₁₋₄-Alkylrest bedeutet und der andere einen C₁₋₄-Alkylrest darstellt; oder
R₃ und R₄ zusammen eine C₂₋₅-Polymethylengruppe ergeben; entweder R₅ ein Wasserstoffatom, einen Hydroxyl-, C₁₋₆-Alkoxy- oder C₁₋₇-Acyloxyrest bedeutet; und
R₆ ein Wasserstoffatom bedeutet; oder
R₅ und R₆ zusammengenommen eine Bindung darstellen; entweder R₇ ein Wasserstoffatom, einen gegebenenfalls mit einer Hydroxy-, C₁₋₆-Alkoxy-, C₁₋₆-Alkoxycarbonyl- oder Carboxylgruppe substituierten C₁₋₆-Alkylrest, einen halogensubstituierten C₁₋₆-Alkylrest oder einen C₂₋₆-Alkenylrest; einen Aryl- oder Heteroarylrest, die beide gegebenenfalls substituiert sind mit einer oder mehreren Gruppen oder Atomen ausgewählt aus C₁₋₆-Alkoxy-, Hydroxy-, Halogen-, Trifluormethyl-, Nitro-, Cyano-, C₁₋ ₁₂-Carboxylacyl- oder gegebenenfalls mit ein oder zwei C₁₋₆-Alkylresten substituierte Amino- oder Aminocarbonylgruppe, bedeutet; und
R₈ ein Wasserstoffatom oder einen C₁₋₆-Alkylrest darstellt; oder
R₇ und R₈ zusammengenommen eine C₃-₄-Polymethylengruppe darstellen, oder die Gruppe CH₂(CH₂)ₙ-Z-(CH₂)ₘ-, in der m und n ganze Zahlen von 0 bis 2 sind, so daß m + n den Wert 1 oder 2 annimmt und Z ein Sauerstoff- oder Schwefelatom oder die Gruppe NR₉ bedeutet, in der R₉ ein Wasserstoffatom, einen C₁₋₉-Alkyl-, C₂₋₇-Alkanoyl-, Phenyl-C₁₋₄-alkyl-, Naphthylcarbonyl-, Phenylcarbonyl- oder Benzylcarbonylrest, gegebenenfalls substituiert mit ein oder zwei C₁₋₆-Alkyl-, C₁₋₆-Alkoxy- oder Halogenresten im Phenyl- oder Naphthylring; oder einen mono- oder bicyclischen Heteroarylcarbonylrest darstellt;
X ein Sauerstoff- oder Schwefelatom darstellt; und die Gruppe R₈NCXR₇ sich in trans-Stellung zur R₅-Gruppe befindet, falls R₅ einen Hydroxyl-, C₁₋₆-Alkoxy- oder C₁₋ ₇-Acyloxyrest bedeutet;
und das ACE-Inhibitor-Antibluthochdruckmittel eine Verbindung der Formel (A), (B) oder (C) ist:

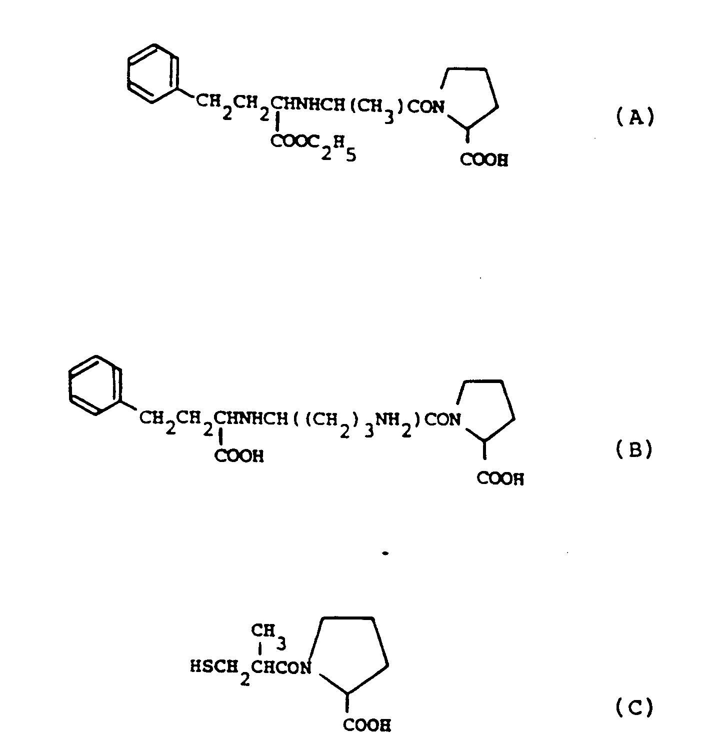

3. Erzeugnis nach Anspruch 2, wobei das Kaliumkanalaktivator-Antibluthochdruckmittel die Formel (I) besitzt, in der Y ein Stickstoffatom oder die Gruppe C-R₁ bedeutet, in der R₁ eine Nitro-, Cyano- oder C₁₋₃-Alkylcarbonylgruppe darstellt und R₂ ein Wasserstoffatom ist.

4. Erzeugnis nach Anspruch 2 oder 3, wobei R₃ und R₄ jeweils Methylgruppen sind.

5. Erzeugnis nach einem der Ansprüche 2, 3 oder 4, wobei R₇ und R₈ zusammengenommen eine C₃₋₄-Polymethylengruppe oder die Gruppe CH₂(CH₂)ₙ-Z-(CH₂)ₘ- bedeuten, in der m, n und Z gemäß Anspruch 2 definiert sind; oder R₈ ein Wasserstoffatom oder eine Methylgruppe darstellen und R₇ eine Ethyl- oder Methylgruppe, ein Wasserstoffatom oder eine gegebenenfalls mit ein, zwei, drei oder vier Gruppen oder Atomen, ausgewählt aus der Methyl-, Methoxy-oder Hydroxylgruppe, dem Fluor- oder Chloratom, substituierte Phenylgruppe bedeutet.

6. Erzeugnis nach Anspruch 5, wobei die Verbindung der Formel (I) (+)-6-Cyano-3,4-dihydro-2,2-dimethyl-trans-4-(2-oxo-1-pyrrolidinyl)-2H-benzo[b]pyran-3-ol ist.

7. Arzneimittel, umfassend ein Kaliumkanalaktivator-Antibluthochdruckmittel und ein ACE-Inhibitor-Antibluthochdruckmittel gemäß einem der Ansprüche 1 bis 6 in Kombination mit einem pharmazeutisch verträglichen Träger.

8. Mittel nach Anspruch 7, wobei das Kaliumkanalaktivator-Antibluthochdruckmittel Pinacidil oder eine Verbindung der Formel (I) gemäß Anspruch 1 oder ein pharmazeutisch verträgliches Salz davon ist und das ACE Inhibitor-Antibluthochdruckmittel eine Verbindung der Formel (A), (B) oder (C) gemäß Anspruch 1 ist.

9. Mittel nach Anspruch 8, enthaltend 0,5 bis 25 mg einer Verbindung der Formel (A), (B) oder (C) je mg Pinacidil oder einer Verbindung der Formel (I) oder eines pharmazeutisch verträglichen Salzes davon.

10. Verwendung eines Erzeugnisses oder Arzneimittels nach einem der Ansprüche 1 bis 9 zur Herstellung eines Kombinationspräparats zur gleichzeitigen, getrennten oder aufeinanderfolgenden Verwendung bei der Bluthochdrucktherapie.

11. Erzeugnis oder Arzneimittel nach einem der Ansprüche 1 bis 10 zur Verwendung bei der Behandlung von Bluthochdruck.

## Patentansprüche (Patentansprüche für folgende(n) Vertragsstaat(en): ES, GR)

1. Verfahren zur Herstellung eines Arzneimittels, umfassend ein Kaliumkanalaktivator-Antibluthochdruckmittel und ein ACE-Inhibitor-Antibluthochdruckmittel und einen pharmazeutisch verträglichen Träger, welches das Vermischen der Wirkstoffe und des Trägers bei nicht-extremer Temperatur von 0 bis 100°C und nicht-extremem Druck von 0,1 bis 10 Atmosphären, vorzugsweise bei Raumtemperatur und Atmosphärendruck umfaßt.

2. Verfahren nach Anspruch 1, wobei das Kaliumkanalaktivator-Antibluthochdruckmittel Pinacidil oder eine Verbindung der Formel (I) ist oder ein pharmazeutisch verträgliches Salz davon, 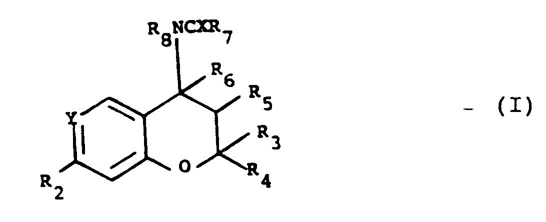 in der
entweder Y ein Stickstoffatom und R₂ ein Wasserstoffatom ist; oder
Y die Gruppe C-R₁ bedeutet,
wobei entweder einer der Reste R₁ und R₂ ein Wasserstoffatom ist und der andere einen C₁₋₆-Alkylcarbonyl-, C₁₋₆-Alkoxycarbonyl-, C₁₋₆-Alkylcarbonyloxy-, C₁₋₆-Alkylhydroxymethyl-, Nitro-, Cyano-, Chlor-, Trifluormethyl-, C₁₋₆-Alkylsulfinyl-, C₁₋₆-Alkylsulfonyl-, C₁₋₆-Alkoxysulfinyl-, C₁₋₆-Alkoxysulfonyl-, C₁₋₆-Alkylcarbonylamino-, C₁₋₆-Alkoxycarbonylamino-, C₁₋₆-Alkylthiocarbonyl-, C₁₋₆-Alkoxythiocarbonyl-, C₁₋₆-Alkylthiocarbonyloxy-, 1-Mercapto-C₂₋₇-alkyl-, Formyl- oder Aminosulfinyl-, Aminosulfonyl- oder Aminocarbonylrest, wobei die Aminogruppe gegebenenfalls mit einem oder zwei C₁₋₆-Alkylresten substituiert ist, oder einen C₁₋₆-Alkylsulfinylamino-, C₁₋₆-Alkylsulfonylamino-, C₁₋₆-Alkoxysulfinylamino- oder C₁₋₆-Alkoxysulfonylaminorest oder einen endständig mit einer C₁₋₆-Alkylcarbonyl-, Nitro- oder Cyanogruppe substituierten Ethylenylrest oder die Gruppe -C(C₁₋₆-Alkyl)NOH oder -C(C₁₋₆-Alkyl)NNH₂ bedeutet;
oder einer der Reste R₁ und R₂ eine Nitro-, Cyano- oder C₁₋₃-Alkylcarbonylgruppe bedeutet und der andere eine Methoxy- oder eine gegebenenfalls mit einem oder zwei C₁₋₆-Alkyl- oder mit einem C₂₋₇-Alkanoylrest substituierte Aminogruppe ist;
entweder einer der Reste R₃ und R₄ Wasserstoff oder einen C₁₋₄-Alkylrest bedeutet und der andere einen C₁₋₄-Alkylrest darstellt; oder
R₃ und R₄ zusammen eine C₂₋₅-Polymethylengruppe ergeben; entweder R₅ ein Wasserstoffatom, einen Hydroxyl-, C₁₋₆-Alkoxy- oder C₁₋₇-Acyloxyrest bedeutet; und
R₆ ein Wasserstoffatom bedeutet; oder
R₅ und R₆ zusammengenommen eine Bindung darstellen; entweder R₇ ein Wasserstoffatom, einen gegebenenfalls mit einer Hydroxy-, C₁₋₆-Alkoxy-, C₁₋₆-Alkoxycarbonyl- oder Carboxylgruppe substituierten C₁₋₆-Alkylrest, einen halogensubstituierten C₁₋₆-Alkylrest oder einen C₂₋₆-Alkenylrest; einen Aryl- oder Heteroarylrest, die beide gegebenenfalls substituiert sind mit einer oder mehreren Gruppen oder Atomen ausgewählt aus C₁₋₆-Alkoxy-, Hydroxy-, Halogen-, Trifluormethyl-, Nitro-, Cyano-, C₁-₁₋₂-Carboxylacyl- oder gegebenenfalls mit ein oder zwei C₁₋₆-Alkylresten substituierte Amino- oder Aminocarbonylgruppe, bedeutet; und
R₈ ein Wasserstoffatom oder einen C₁₋₆-Alkylrest darstellt; oder
R₇ und R₈ zusammengenommen eine C₃-₄-Polymethylengruppe darstellen, oder die Gruppe CH₂(CH₂)ₙ-Z-(CH₂)ₘ-, in der m und n ganze Zahlen von 0 bis 2 sind, so daß m + n den Wert 1 oder 2 annimmt und Z ein Sauerstoff- oder Schwefelatom oder die Gruppe NR₉ bedeutet, in der R₉ ein Wasserstoffatom, einen C₁₋₉-Alkyl-, C₂₋₇-Alkanoyl-, Phenyl-C₁₋₄-alkyl-, Naphthylcarbonyl-, Phenylcarbonyl- oder Benzylcarbonylrest, gegebenenfalls substituiert mit ein oder zwei C₁₋₆-Alkyl-, C₁₋₆-Alkoxy- oder Halogenresten im Phenyl- oder Naphthylring; oder einen mono- oder bicyclischen Heteroarylcarbonylrest darstellt;
X ein Sauerstoff- oder Schwefelatom darstellt; und die Gruppe R₈NCXR₇ sich in trans-Stellung zur R₅-Gruppe befindet, falls R₅ einen Hydroxyl-, C₁₋₆-Alkoxy- oder C₁-₇-Acyloxyrest bedeutet;
und das ACE-Inhibitor-Antibluthochdruckmittel eine Verbindung der Formel (A), (B) oder (C) ist:

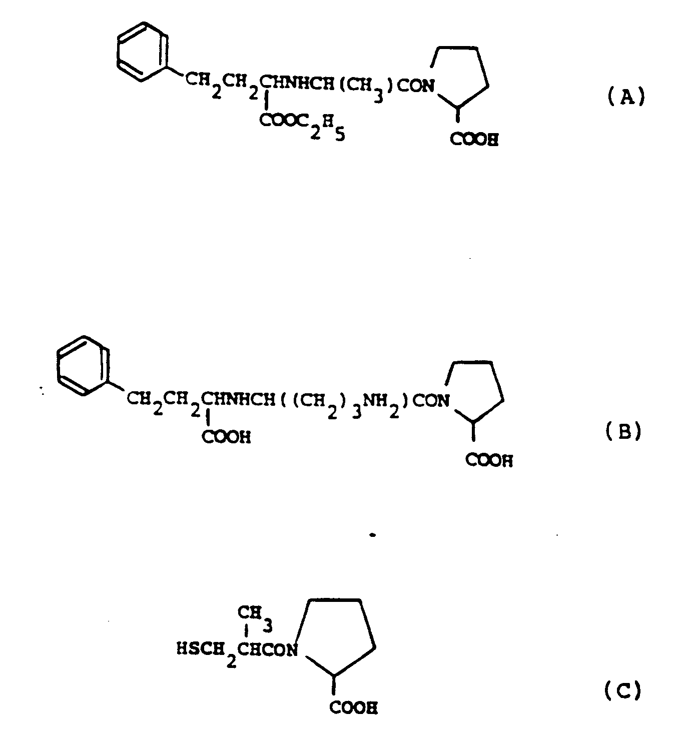

3. Verfahren nach Anspruch 2, wobei das Kaliumkanalaktivator-Antibluthochdruckmittel die Formel (I) besitzt, in der Y ein Stickstoffatom oder die Gruppe C-R₁ bedeutet, in der R₁ eine Nitro-, Cyano- oder C₁₋₃-Alkylcarbonylgruppe darstellt und R₂ ein Wasserstoffatom ist.

4. Verfahren nach Anspruch 2 oder 3, wobei R₃ und R₄ jeweils Methylgruppen sind.

5. Verfahren nach einem der Ansprüche 2, 3 oder 4, wobei R₇ und R₈ zusammengenommen eine C₃₋₄-Polymethylengruppe oder die Gruppe CH₂(CH₂)ₙ-Z-(CH₂)ₘ- bedeuten, in der m, n und Z gemäß Anspruch 2 definiert sind; oder R₈ ein Wasserstoffatom oder eine Methylgruppe darstellen und R₇ eine Ethyl- oder Methylgruppe, ein Wasserstoffatom oder eine gegebenenfalls mit ein, zwei, drei oder vier Gruppen oder Atomen, ausgewählt aus der Methyl-, Methoxyoder Hydroxylgruppe, dem Fluor- oder Chloratom, substituierte Phenylgruppe bedeutet.

6. Verfahren nach Anspruch 5, wobei die Verbindung der Formel (I) (±)-6-Cyano-3,4-dihydro-2,2-dimethyl-trans-4(2-oxo-1-pyrrolidinyl)-2H-benzo[b]pyran-3-ol ist.

7. Verfahren nach Anspruch 2, wobei das Mittel 0,5 bis 25 mg einer Verbindung der Formel (A), (B) oder (C) je mg Pinacidil oder einer Verbindung der Formel (I) oder eines pharmazeutisch verträglichen Salzes davon enthält.

8. Verwendung eines pharmazeutischen Erzeugnisses, umfassend ein Kaliumkanalaktivator-Antibluthochdruckmittel und ein ACE-Inhibitor-Antibluthochdruckmittel bei der Herstellung eines Kombinationspräparats zur gleichzeitigen, getrennten oder aufeinanderfolgenden Verwendung bei der Bluthochdrucktherapie.
